# EUROPEAN PATENT SPECIFICATION

(11) **EP 2 702 983 B1**
(45) Date of publication and mention of the grant of the patent: **10.10.2018**
(21) Application number: 13179624.5
(22) Date of filing: 07.08.2013
(51) Int. Cl.: A61K 9/00, A61K 47/10, A61K 9/06, A61K 47/44, C11D 3/48, A61K 31/4425

(54) **Process for the preparation of a bispyridiniumalkane-containing semisolid preparation**
Verfahren zur Herstellung einer bispyridiniumalkanhaltigen halbfesten Zubereitung
Procédé pour la préparation d'une préparation semi-solide contenant du bispyridiniumalkane

(30) Priority: 31.08.2012 DE 102012215511
(43) Date of publication of application: 05.03.2014
(73) Proprietor: L'AIR LIQUIDE, SOCIETE ANONYME POUR L'ETUDE ET L'EXPLOITATION DES PROCEDES GEORGES CLAUDE, 75007 Paris (FR); SCHÜLKE & MAYR GMBH, 22851 Norderstedt (DE)
(72) Inventor: Behrends, Sabine, 25482 Appen (DE); Dettmann, Andreas, 22175 Hamburg (DE); Radischat, Nadine, 22297 Hamburg (DE); Hahn, Mona, 21335 Lüneburg (DE)
(74) Representative: Conan, Philippe Claude

(56) References cited:
- US-A1- 2008 221 165
- US-A1- 2011 091 551
- NILS-OLAF HÜBNER ET AL: "Antibiotikafreie Sanierung von MRSA-positivem Personal", GMS KRANKENHAUSHYGIENE INTERDISZIPLINÄR, vol. 4, no. 2, 16 December 2009 (2009-12-16), pages 1/4-4/4, XP55089222, DOI: 10.3205/dgkh000129

## Description

The present invention relates to a process for the preparation of octenidine dihydrochloride-containing multiphase semisolid preparation in the form of an use preparation which is an ointment. In the process, octenidine dihydrochloride is pre-dissolved in glycerol, 1,2-propylene glycol or mixture thereof. The use preparation is applied in particular to the nasal mucous membrane to control multi-resistant pathogens and Gram-negative microorganisms. Furthermore, the invention relates to a kit which comprises the semisolid preparation (in particular the use preparation).
The increase in the number of patients colonized by, or infected with, multiresistant *Staphylococcus aureus* (MRSA) strains is one of the big clinical and epidemiological problems worldwide. *Staphylococcus aureus* are bacteria which belong to the normal flora of the human skin and mucous membranes. Most frequently, it is the mucous membranes of the pharynx and of the vestibule of the nose which are colonized. Approximately 20% of the population in the Federal Republic of Germany are permanently colonized, and 60% intermittently colonized, with staphylococci. Approximately 0.3 to 5% of the population in Germany are MRSA carriers.
The staphylococci are transmitted predominantly via direct contact, in the field of medicine mostly via the hands of patients and staff. The innocuous microbe on the skin may, via injuries, operations, other invasive medical procedures or disturbances of the immune system, penetrate into the human body and cause, inter alia, infections of wounds, soft parts or bones, endocarditis, pneumonia or life-threatening sepsis.

Ointments (Latin: unguenta) are specific spreadable semisolid preparations which are intended to be employed by application to the skin or rubbing onto the skin. They consist of one or more ointment bases. As a rule, ointments are virtually anhydrous. They have advantages over other pharmaceutical forms in particular when applied to the mucous membrane area, such as, for example, the nose, since they do not dry out the nose, and adhere well to the set application, in particular when the ointment is what is known as an absorption base. An absorption base is a system which is capable of adsorbing water. As a rule, this is achieved by adding specific emulsifiers. Drug substances may be present in the base in dissolved form (solution ointments) or in suspended form (suspension ointments) (Rudolf Voigt, Pharmazeutische Technologie [Pharmaceutical Technology], 11th edition, page 383). In contrast, powders are unsuitable as pharmaceutical forms in the nasal mucosa area because they dry out the nose.

Preparations which are used for removing MRSA from the nasopharyngeal zone are those based on mupirocin (pseudomonic acid A).

EP 0 231 621 A describes a topical pharmaceutical containing pseudomonic acid and its salts or esters. WO 95/10999 A discloses a composition which comprises a therapeutic agent in a cream base, the cream base comprising 45 to 60% by weight of mineral oil, 5 to 15% by weight of fatty alcohols or fatty esters, 4 to 8% by weight of polyoxyethylene ether or ester surfactant and 20 to 35% by weight of water. WO 98/05313 A relates to a composition which contains mupirocin and chlorhexidin. WO 99/54048 A describes a process and a device for micronizing specially preferred crystalline calcium mupirocin dehydrate.

However, infections with Gram-negative bacteria, anaerobic bacteria and *Enterococcus* species cannot be treated with mupirocin. The use of antibiotics such as mupirocin involves the risk of the bacteria developing resistance. The prolonged use of Turixin may therefore mean that the therapy fails.

The alternative approach in the case of mupirocin resistance is to control MRSA with preparations based on 1.25% povidone-iodine (PVP-iodine). However, the use of PVP-iodine may result in allergies. Moreover, PVP-iodine may be absorbed. Absorption is a function of the area over which PVP-iodine is used, the amount applied and the use frequency. Depending on the applied dose or the exposed area, iodine concentrations which are critical for the hyperthyroid thyroid and, under certain circumstances, even for a euthyroid thyroid can be reached. When using iodophores, therefore, the contraindications hyperthyroid thyroid diseases and hypersensitivity to iodine must be taken into consideration.

It is known from N. Sloot, J. Siebert and U. Höffler (Zbl. Hyg. Umweltmed. 202 (1999), p. 513-523) that a full body wash with an aqueous solution which contains octenidin dihydrochloride (a bispyridiniumalkane, hereinbelow abbreviated to "octenidin") and phenoxyethanol may form part of an MRSA therapy, to be precise in combination with the use of mopirocin in the nose. The full body wash may have side effects, more precisely reddening of the skin (see also U. Rohr, C. Mueller, M. Wilhelm, G. Muhr and S. Gatermann (Journal of Hospital Infection (2003) 54, p. 305-309). There was therefore also a need for a preparation and a method for controlling MRP without these side effects.

WO 2006/029255 A2 describes antimicrobial compositions and processes for their preparation. The compositions may be applied to mucous membranes. The intended cationic antiseptic is, among others, octenidin. A process for the preparation of a formulation containing octenidin is, however, not described in WO 2006/029255.

DE 10 2010 044 785 A1 discloses preparations which are based on a microemulsion with an octenidin content as coemulsifier. The preparations are employed as deodorant formulations, with the octenidin improving the transparency of the microemulsions. DE 10 2010 044 787 A1 maintains that octenidin is not effective in microemulsions with a low active substance content, and that either the active substance content may be increased or that, according to the teaching of DE 10 2010 044 787 A1, the product should be formulated as a macroemulsion.

DE 10 2005 045 145 A1 furthermore discloses semisolid preparations (such as ointments) which contain octenidin dihydrochloride (a bispyridiniumalkane, hereinbelow abbreviated to octenidin), for example in the form of an ointment. In principle, bispyridiniumalkanes would be suitable as active substances for controlling MRSA. However, the ointment base in DE 10 2005 045 145 A1 contains a mixture of wool wax alcohols. For example, Eucerit® (Beiersdorf AG, Hamburg, Federal Republic of Germany) is a mixture of aliphatic alcohols (alkanols with chain lengths of from C₁₈ to C₂₀, diols with chain lengths of from C₁₆ to C₂₆) and sterols, with a cholesterol content of at least 30%. Wool wax alcohols occasionally contain pesticide residues and may trigger allergies and are therefore undesired.

The successful and long-term sanitation of the body in the case of MRSA infection is only successful when an overall concept is employed for the antiseptic treatment (cf. N.-O. Hübner et al., Sanierung von MRSA-positiven Patienten [Sanitation of MRSA-positive Patients], in Arzneimittel-therapie 2009; 27:171 to 7). The octenidin nasal ointment concentrate described therein comprises 2.0% by weight of octenidin, 60.0% by weight of wool wax alcohols, 5.0% by weight of cetylstearyl alcohol and 33.0% by weight of soft liquid paraffin. To prepare the ointment, one (1) part by weight of the concentrate is formulated together with 9 parts by weight of Vaseline. Octenilin wound gel comprises 0.05% by weight of octenidin, 9.95% by weight of propylene glycol, 2.5% by weight of hydroxyethylcellulose and 87.5% by weight of fully-demineralized water.

The bispyridiniumalkane used in the formulation of the present invention is octenidine dihydrochloride.

Therefore it was an object of the present invention to provide an antiseptic preparation based on bispyridiniumalkane, by means of which MRSA can be controlled effectively, in particular also in the nose. It is intended that the preparation be well tolerated, i.e. no hypersensitivity reactions on the nasal mucosa should occur (in other words, the preparation should not necessarily contain wool wax alcohols). It is intended that the preparation can be formulated as desired, in other words, including as a lipophile preparation, if desired. Furthermore, it should allow control of a wide range of Gram-negative microbes and multi-resistant pathogens (MRPs), in particular MRSA and ESBL producers. Furthermore, it is intended that the preparation be capable of absorbing water from the nasal secretion, which is important for physiological reasons. A particular challenge was furthermore to formulate the system with a low water content. This is important for the system to still be capable of absorbing nasal secretion while still adhering well to the nasal mucosa.
Another object was therefore also to provide a possibility of incorporating a relatively hydrophilic active substance (bispyridiniumalkane) into an (optionally lipophilic) preparation in such a manner that the preparation has a sufficient antimicrobial activity. Typically, one would, for this purpose, incorporate the active substance into an ointment base in finely-suspended form. In an example of a formula with bispyridiniumalkane (octenidin), which had been micronized down to as little as 10 µm, however, preliminary experiments have shown that the antimicrobial activity of a preparation which contains bispyridiniumalkane which had been incorporated in micronized form is insufficient.

Surprisingly, the object was achieved by predissolving the bispyridiniumalkane in a component of the preparation.

It has thus emerged that the abovementioned and further objects are achieved by an antiseptic semisolid preparation into which the bispyridiniumalkane, completely dissolved in solvent, has been incorporated. When applied to the nasal mucosa, this semisolid preparation has at least an equally good activity against MRPs as the known mupirocin-based preparation known and sold as Turixin®. It was surprising that the semisolid preparations according to the invention have a good activity because, as has been mentioned hereinabove, it is usually assumed that suspension ointments have a better activity than solution ointments. Thus, Bauer-Frömming-Führer describe in Lehrbuch der Pharmazeutischen Technologie [Text Book of Pharmaceutical Technology], 8th edition, 2006, p. 288, that active substances from a suspension ointment are usually absorbed better by the skin than those from a solution ointment, which the authors attribute to the presence of more favourable data for the distribution coefficient. Furthermore, it is claimed that a more constant penetration rate can be achieved from suspension ointments since undissolved active substance continues to dissolve in the base to the same extent as dissolved active substance penetrates into the skin.

The invention therefore relates to a process for preparing a multiphase semisolid preparation in the form an ointment which contains octenidine dihydrochloride, in which:
i)Octenidine dihydrochloride is dissolved in a solvent selected from among 1,2-propylene glycol, glycerol and mixtures thereof, so as to prepare a solution of 0.01% to 5% by weight of octenidine dihydrochloride, based on the weight of the solution (phase I),
ii) an emulsifier with an HLB<8 is provided together with a lipid phase (phase II),
iii) phases I and II are warmed separately at a temperature from 50°C to 100°C,
iv) phase I is incorporated into phase II, such that the weight ratio between the phase I and the phase II amounts from 1:99 to 20:80 and
v) if appropriate, the mixture is homogenized.

In this context, the expression "solution" with respect to step a) of the process according to the invention relates to the preparation of a homogeneous liquid mixture, i.e. the bispyridiniumalkane is dissolved completely in the solvent (or solvent mixture).

### Bispyridiniumalkane

The expression bispyridiniumalkane comprises the bis[4-(substituted-amino)-1-pyridinium]alkanes of the general formulae (I) or (II) disclosed in DE 27 08 331 C2 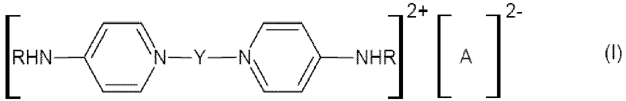 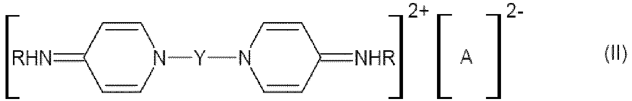 in which
Y is an alkylene group having 4 to 18 carbon atoms,
R represents an alkyl group having 6 to 18 carbon atoms or a cycloalkyl group having 5 to 7 carbon atoms or the phenyl radical which is substituted by a halogen atom, and
A is an anion or several anions.
Strictly speaking, the abovementioned definition of A applies to monovalent and divalent anions, but A may of course also be a polyvalent anion, for example phosphate or orthosilicate. Furthermore, the expression bispyridiniumalkane comprises the various prototropes of the compounds of the formula (I) such as, for example, as disclosed in DE 196 47 692 A1.

However, in all embodiments of the invention the bispyridiniumalkane is octenidine dihydrochloride (R = n-octyl, Y = n-decenyl; A = 2 × Cl, hereinbelow "octenidin").
Preferred amounts of the bispyridiniumalkane in the semisolid preparation according to the invention are from 0.01 to 1.0% by weight, preferably from 0.02 to 0.5% by weight, more preferably from 0.03 to 0.3% by weight, even more preferably from 0.04 to 0.1% by weight, such as, for example, 0.05% by weight.

Preferably, the temperature in step a) is from 50 to 100°C, in particular from 55 to 90°C, for example from 60 to 85°C, such as from 65 to 80°C.
The solvent for the bispyridiniumalkane in step a) is selected from 1, 2-propylene glycol, glycerol and mixtures thereof. It is clear to a person skilled in the art that the solvent must be compatible with the further formulation components so that the desired semisolid preparation can be formulated.
The concentration of the bispyridiniumalkane of the solution prepared in step a) amounts to 0.01 to 5% by weight, preferably 0.02 to 2% by weight, more preferably 0.05 to 1% by weight and in particular 0.1 to 0.5% by weight, based on the weight of the solution.

The procedure is followed in the process according to the invention such that, in step b), the further usual constituent or the optionally further usual constituents is/are brought to a temperature in the range of from 50 to 100°C (preferably from 60 to 90°C, in particular from 70 to 80°C) and the solution of the bispyridiniumalkane from step a) is then added to the constituent(s), with the solution preferably being added at the same temperature. Here, the solution of step a) is preferably first brought to the temperature of the further constituent(s) and then added at this (same) temperature as the remaining constituent(s) .

In the process according to the invention, a procedure is followed in which
i) the solution of the bispyridiniumalkane is prepared (phase I),
ii) at least part of at least one of the further customary constituents of a semisolid preparation is provided (phase II),
iii) phases I and II are warmed separately,
iv) phase I is incorporated into phase II, and
v) if appropriate, the mixture is homogenized.
Preferred semisolid preparations according to the invention have good spreadability. The spreadability is determined by the following method:
a) 1.0 g of the test substance (in the case of emulsions 0.5 g) is applied to a sheet of glass (12 × 12 cm),
b) a second sheet of glass is superimposed precisely on the first sheet of glass,
c) after a brief settling time of the second sheet, the edges of the sheets are fixed so that displacing of the sheets as a result of the subsequent weighting is prevented, and
d) the upper sheet is weighted for 10 seconds with a 350 g weight.
Then, the extent of the oval compressed ointment or emulsion area between the two sheets (viewed from above) is determined at two positions (at a 90° angle to each other). The spreadability is then given in mm. The higher this mean, the better the spreadability and therefore the distribution.

The semisolid preparation is in the form of a use preparation. The use preparation is prepared in accordance with the process according to the invention and it contains from 0.001 to 0.1% by weight of bispyridiniumalkane based on the total weight of the preparation.
In other words, the invention in further embodiments is a use preparation (in the form of an ointment) which contains from 0.001 to 0.1% by weight of bispyridiniumalkane and whose preparation includes the dissolving of the bispyridiniumalkane in solvent. In this context, it is preferred that semisolid preparations according to the invention (ointments) comprise less than 10% by weight of water, based on their weight. A preferred preparation contains less than 5% by weight of water, more preferably less than 3% by weight of water, such as less than 2% by weight of water. An especially preferred preparation is essentially free from added water, i.e. it contains only the small amount of water which the use of remaining constituents of the semisolid preparation entails. Preferred preparations are free from wool wax alcohols.
It is furthermore preferred that the preparation according to the invention comprises less than 20% by weight of solvent based on the total weight of the preparation.
Especially preferred are semisolid preparations according to the invention which are free from antibiotics.
In addition, preferred semisolid preparations are those which contain none of the usual thickeners such as cellulose derivatives, xanthans, pectins, polyacrylates, gelatine or agar.
The semisolid preparation (in particular use preparation, such as ointment) is present as a multi-phase embodiment. As a multiphase (preferably two-phase) embodiment, it also includes a lipid phase besides the phase in which the bispyridiniumalkane has been predissolved. Constituents of the lipid phase are preferably Vaseline, liquid paraffin, triglycerides and mixtures thereof. Further lipids of the lipid phase may be, for example, beeswax, hard fats, hard waxes, hard paraffin and pig fat.

The weight ratio between the solution prepared in step a) and the further constituents added in step b) (preferably a lipid phase) is, in this context, preferably from 1:99 to 20:80, preferably from 2:98 to 15:85, such as from 5:95 to 10:90.
The solvent amounts preferably to from 1 to 15% by weight, but preferably 7 to 12% by weight, especially preferably approximately 10% by weight, based on the weight of the semisolid preparation.
Furthermore, it is possible to formulate an advantageous semisolid preparation according to the invention with the aid of emulsifiers, in particular emulsifiers with an HLB value (HLB = hydrophilic-lipophilic balance) of < 8. The emulsifier is incorporated together with the constituents added in step b), and no emulsifier is introduced into the phase prepared in step a).
Examples of emulsifiers are selected from among (partial) esters of (poly)glycerol, for example glycerol (mono, di, tri) [adipate, alkanoate (C₈, C₁₀ and C₁₈), isostearate], triglycerol diisostearate, polyglyceryl 2-dipolyhydroxystearate, glyceryl oleate, sorbitan laurate and cetyl stearyl acetate.

In all embodiments of the invention, it is preferred that the semisolid preparation (in particular use preparation, such as ointment) does not comprise any C₁-to C₄-alkanols.

The semisolid preparation according to the invention (in particular use preparation, such as ointment) may additionally comprise antioxidant. Examples of antioxidants are disclosed in DE 10 2008 011 691 A1. Antioxidants which are preferably present and which act in accordance with the invention as stabilizers for bispyridiniumalkanes are acetylcystein, 3-tert-butyl-4-hydroxyanisole, 2,6-di-tert-butyl-p-cresol, tert-butylhydroquinone, caffeic acid, chlorogenic acid, cystein, cystein hydrochloride, decylmercaptomethylimidazole, diamylhydroquinone, di-tert-butylhydroquinone, dicetyl thiodipropionate, digalloyl trioleate, dilauryl thiodipropionate, dimyristyl thiodipropionate, dioleyl tocopheryl methylsilanol, disodium rutinyl disulphate, distearyl thiodipropionate, ditridecyl thiodipropionate, dodecyl gallate, erythorbic acid, ethyl ferulate, ferulic acid, hydroquinone, p-hydroxyanisole, hydroxylamine hydrochloride, hydroxylamine sulphate, isooctyl thioglycolate, kojic acid, madecassicoside, methoxy-PEG-7-rutinyl succinate, nordihydroguaiaretic acid, octyl gallate, phenylthioglycolic acid, phloroglucinol, propyl gallate, rosmarinic acid, rutin, sodium erythorbate, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocophersolan, tocopherol (for example vitamin E) and its derivatives (for example vitamin E derivatives such as vitamin E acetate, vitamin E linoleate, vitamin E nicotinate and vitamin E succinate), o-tolylbiguanide, tris(nonylphenyl) phosphite, dexpanthenol, alpha-hydroxycarboxylic acids (for example glycol acid, lactic acid, mandelic acid) and their salts, p-hydroxybenzoic esters (for example their methyl, ethyl, propyl or butyl esters), dimethyloldimethylhydantoin, N-acylamino acids and their salts (for example N-octanoylglycin, Lipacide C8G), ascorbic acid and hinoktiol.

The tocopherols are particularly effective antioxidants according to the invention. Furthermore, the tocopherols are particularly desirable antioxidants with regard to the uses of the preparations according to the invention, which are associated with stringent legal provisions and with toxicity tests, in the preparation of cosmetics and pharmaceuticals.

Tocopherols are present in vegetable oils. Particularly rich in tocopherols are seed oils from soya, wheat, maize, rice, cotton, lucern and nuts, fruits and vegetables such as raspberries, legumes, fennel, paprika and celery.

The physiological action of tocopherols is based on their properties as free-radical scavengers. Thus, the tocopherols, when they are used according to the invention as antioxidants and thus also pass in small amounts into the preparations which contain bispyridiniumalkane, can themselves act as physiologically active antioxidants even in the cell membrane and in lipoproteins. Alpha-tocopherol (vitamin E, antisterility factor) is the most physiologically effective and most widespread natural tocopherol.

Although the tocopherols used may be of synthetic origin, tocopherols of natural origin may be used. It is possible to use sterically uniform enantiomers or enantiomer mixtures of tocopherols; accordingly, for the derivatization to acetate, succinate, linoleate or nicotinate, it is possible to use tocopherols of natural and/or synthetic origin and sterically uniform enantiomers or mixtures of tocopherols (in particular alpha-tocopherol).

Stabilizers employed in accordance with the invention are preferably selected from among α-tocopherol, 2,6-di-tert-butyl-4-methylphenol (BHT), tocopherol acetate, 2-tert-butyl-4-hydroxyanisole and/or 3-tert-butyl-4-hydroxyanisole (BHA), dodecyl gallate and ascorbic acid. Preferred stabilizers are vitamin E, vitamin E derivatives, BHA, BHT and alkyl gallate, or combinations of these substances, in particular α-tocopherol and BHT.

Typically, the preparation according to the invention is prepared in such a way that the bispyridiniumalkane is dissolved in the solvent. Thereafter, the further constituents which may be present are added, for example the constituents of the lipid phase.
For example, the constituents of the lipid phase and any emulsifiers may be molten together, and the solution of the bispyridiniumalkane may be added to the melt.
An especially preferred use of the preparations according to the invention preparations is the antiseptic treatment of the nose, especially preferably against MRPs (for example MRSA (methicillin-resistant *Staphylococcus aureus*) and other multi-resistant microbes, such as ESBL).
Accordingly, a further aspect of the invention relates to the preparation for use for controlling Gram-negative microbes and MRPs, specifically MRSA and ESBL, in particular ESBL or MRSA. It is preferred to apply the preparation to the nasal mucosa.

Finally, the invention relates to a kit for controlling Gram-negative microbes and MRPs, in particular MRSA and ESBL. A kit according to the invention for the complete semitation of MRP carriers comprises, besides the semisolid preparation according to the invention (the use formulation in the form of an ointment) at least one of the following:
(1) a rinse for the oropharyngeal cavity,
(2) a full-body wash lotion,
(3) one or more towelettes for applying the use preparation, such as ointment and/or the components (1) and/or (2),
(4) a surface disinfectant for disinfecting the surroundings of disinfected patients,
(5) a hand disinfectant and
(6) use instructions for the MRP sanitation.

A preferred rinse for the oropharyngeal cavity (1) is disclosed in DE 10 2006 051 891 A1 and DE 10 2008 011 691 A1. It is preferred that this rinse comprises the following:
(a) octenidin in an amount of from 0.05 to 0.3% by weight, such as 0.1 to 0.2% by weight,
(b) glycerol (85% strength) in an amount of from 0.3 to 1.0% by weight, such as, for example, 0.5% by weight,
(c) sodium gluconate in an amount of from 0.2 to 0.6% by weight, such as, for example, 0.4% by weight,
(d) sucralose in an amount of from 0.05% by weight to 0.2% by weight, such as 0.1% by weight,
(e) nonionic surfactant (in particular macrogolglycerolhydroxystearate-40 EO) in an amount of from 1% by weight to 3% by weight, such as, for example, 1.75% by weight,
(f) aroma in an amount of from 0.3% by weight to 1.0% by weight, such as, for example, 0.5% by weight,
(g) BHT in an amount of from 0% by weight to 0.6% by weight, such as 0% by weight to 0.04% by weight,
(h) citric acid in an amount of from 0.05% by weight to 0.2% by weight, such as, for example, 0.1% by weight,
(i) buffer in an amount of from 0.1% by weight to 1.0% by weight, such as 0.2% by weight to 0.8% by weight, and
(j) water to 100% by weight.

Such a rinse (1) is present at a pH of from 5 to 8, such as, preferably, 5.5 to 7.0.

In a further preferred embodiment, the wash lotion (2) contains
(a) octenidin in an amount of from 0.05 to 1.0% by weight, preferably 0.1 to 0.5% by weight, such as, for example, 0.3% by weight,
(b) glycerol (85% strength) in an amount of from 0.3 to 5.0% by weight, preferably 1.0 to 2.0% by weight, such as, for example, 1.3% by weight,
(c) non-ionic surfactant (preferably PEG-7 glyceryl cocoate) in an amount of from 0.5 to 7.0% by weight, preferably 1.0 to 5.0% by weight, such as, for example, 2.5% by weight,
(d) amine oxide (preferably coconut fatty acid amidopropyldimethylamine oxide 35% strength = Rewominox B 204) in an amount of from 5.0 to 60% by weight, preferably from 20 to 35% by weight, such as, for example, 28.6% by weight,
(e) urea derivative (preferably allantoin) in an amount of from 0.05 to 0.5% by weight, preferably from 0.1 to 0.5% by weight, such as, for example, 0.2% by weight,
(f) thickener (preferably hydroxyethylcellulose) in an amount of from 0.1 to 3.0% by weight, preferably from 0.5 to 2.0% by weight, such as, for example, 1.0% by weight,
(g) organic acid (preferably lactic acid) in an amount of from 0.1 to 3.0% by weight, preferably 0.5 to 2.0% by weight, such as, for example, 0.9% by weight, and
(h) water to 100% by weight.

To prepare the wash lotion, the constituents (a) to (e) and (h) are combined and dissolved to form a clear solution. Then, the thickener (f) is sprinkled in, and stirring is continued until a clear viscous solution has formed. Then, the organic acid (g) is added and, if appropriate, the pH is adjusted to from 4.8 to 5.0.

The advantages of the invention can be seen in particular from the examples which follow. Unless otherwise specified, percentages are by weight.

### Examples

Softisan 649 = partial ester of diglycerol with fatty acids of medium chain length (isostearic acid, stearic acid, 1, 2-hydrostearic acid, adipic acid), (CAS 130 905-60-1), Sasol Germany GmbH, Witten, Federal Republic of Germany.

### 1. Experimental method:

Modified experimental method for testing the microbial activity of the MRSA nasal ointment, analogously to: "RAPA: A novel in vitro method to evaluate antibactericidal skin cleansing products" (International Journal of Cosmetic Science 2010, 32, 107-116) .

### Experimental set-up:

A CSA plate (CSA = soybean casein digest agar) was inoculated with 0.1 ml of bacterial suspension and plated until fully dry. Before further processing, the agar plates were left to stand for 30 minutes at room temperature. The test preparations are fully soaked up by the agar.

The test organism used was an MRSA strain from the DSMZ (Deutsche Sammlung von Mikroorganismen und Zellkulturen [German Collection of Microorganisms and Cell Cultures], Braunschweig, Federal Republic of Germany) (*Staphylococcus aureus* ATCC 33592). The initial bacterial count of the bacterial suspensions was adjusted as follows: 2 × 10⁵ CFU/ml.
In each case 0.05 ml of ointment of the formulations hereinbelow were distributed on the inoculated agar plates, using a finger and a sterile glove. The samples were distributed from the middle outwards over the entire plate until the plate was fully wetted (within approximately 10-15 seconds).

### 2. Example A

| **Formulation** | **A1** | **A2** | **A (placebo)** |
|---|---|---|---|
| Octenidin, % | 0.05 | 0.05 | - |
| Glycerol 100%, % | 4.95 | 4.95 | 5.00 |
| Softisan 649, % | 2.00 | 2.00 | 2.00 |
| Vaseline, % | 93.00 | 93.00 | 93.00 |
| Preparation | Octenidin is dissolved in the glycerol, and the solution is then incorporated into Softisan and vaseline | Triturate octenidin with the mixture of Softisan and vaseline, warm glycerol on its own and add | |

Example A2 is a comparative example. Example A (placebo) does not fall in the scope of the claims.

The evaluation is carried out via photographic documentation.

### b) Results:

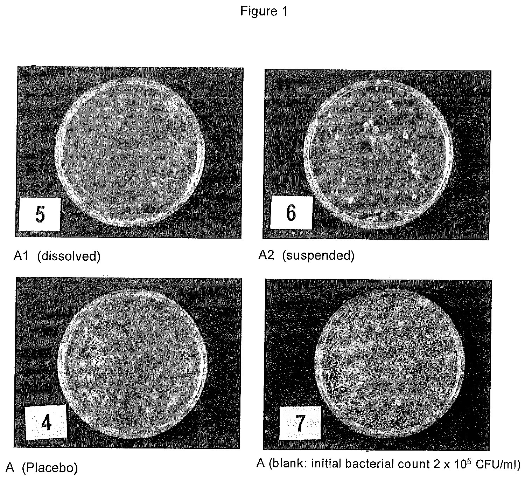

The results are shown in Figure 1.

### Evaluation:

As can be seen from the photographic documentation, predissolving the active substance octenidin dihydrochloride improves the activity of the ointment in comparison with the octenidin-containing suspension ointment.

### 3. Example B

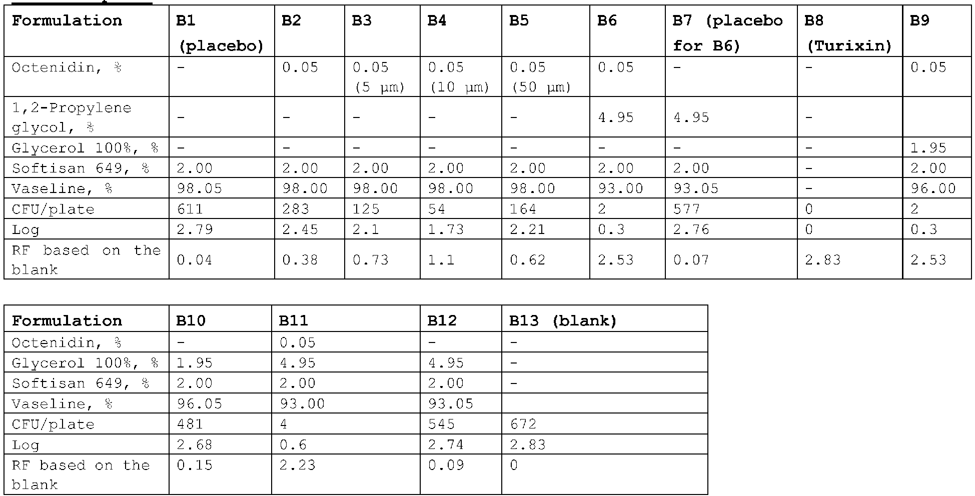

The formulations B1-B5, B7, B8, B10, B12, and B13 are not in the scope of the claims. The amount applied was in each case 0.10 ml.

The evaluation was done via photographic documentation, followed by counting the CFU on each plate and calculating the RF factors based on the blank (plate B13) .

### Results:

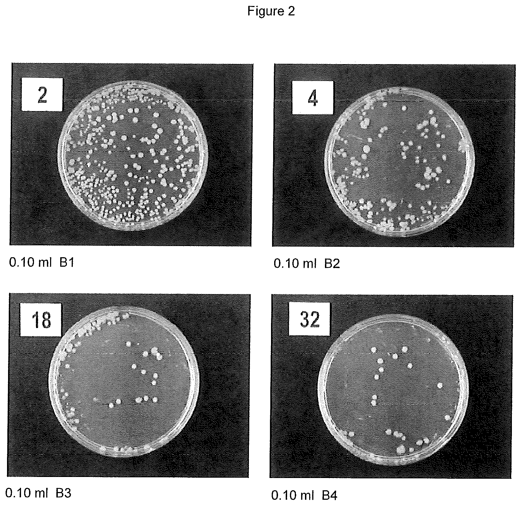

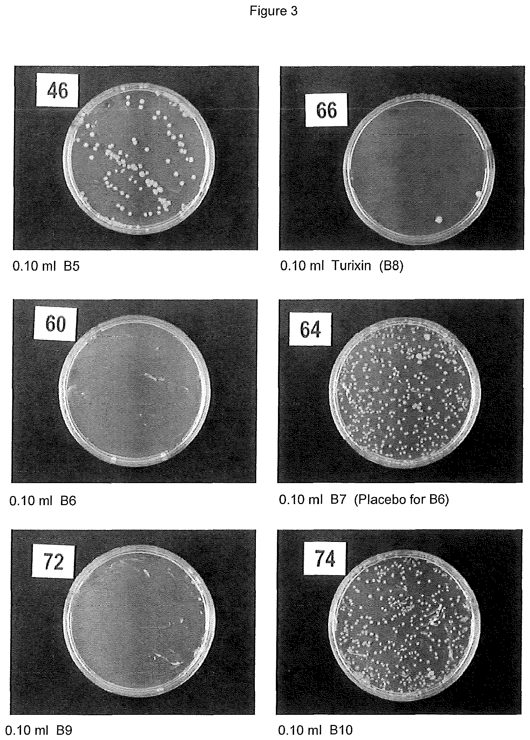

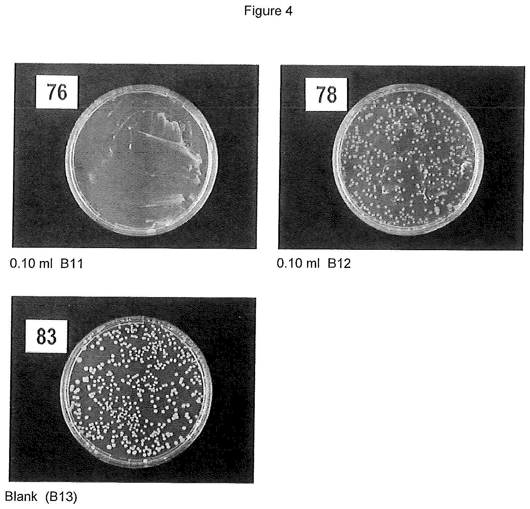

The results are shown in Figures 2 to 4.

### Evaluation:

The Turixin plate shows two microbes, but these are not MRSA, the Turixin plate contains zero (0) microbes. This results in an RF value of 2.83. This RF value is not obtained with the suspension ointments containing octenidin alone (plates B2-B5); the RF values are between 0.38-1.10. The solution ointments generally perform better than the suspension ointments. Formulation B6, which contains 4.95% propylene glycol, has an RF value of 2.53 and therefore approaches the activity of Turixin. Only two microbes grow on the plate. The activity of glycerol-containing formulations (B9 and B11) is good, too; only two and four microbes, respectively, grow. The RF values amount to 2.53 (B9) and 2.23 (B11).

Thus, an approximately equally good activity is obtained with the solution ointments in comparison with Turixin. The activity of the suspension ointments is clearly worse.

## Claims

1. Process for preparing a multiphase semisolid preparation in the form an ointment which contains octenidine dihydrochloride, in which:
i) Octenidine dihydrochloride is dissolved in a solvent selected from among 1,2-propylene glycol, glycerol and mixtures thereof, so as to prepare a solution of 0.01% to 5% by weight of octenidine dihydrochloride, based on the weight of the solution (phase I),
ii) an emulsifier with an HLB<8 is provided together with a lipid phase (phase II),
iii) phases I and II are warmed separately at a temperature from 50°C to 100°C,
iv) phase I is incorporated into phase II, such that the weight ratio between the phase I and the phase II amounts from 1:99 to 20:80 and
v) if appropriate, the mixture is homogenized.

2. Process according to Claim 1, **characterized in that** the concentration of octenidine dihydrochloride in the solution prepared in step i) amounts to 0.02 to 2% by weight, based on the weight of the solution.

3. Process according to one of the preceding claims, **characterized in that** the weight ratio between phase I and phase II amounts to from 2:98 to 15:85.

4. Process according to one of the preceding claims, **characterized in that** the lipid phase comprises Vaseline, liquid paraffin, triglycerides or mixtures thereof and one or more emulsifiers with an HLB value of < 8, selected from among glycerol (mono, di, tri) [adipate, alkanoate (C₈, C₁₀ and C₁₈), isostearate], polyglyceryl esters, sorbitan laurate and cetylstearyl acetate and mixtures thereof.

## Patentansprüche

1. Verfahren zur Herstellung einer mehrphasigen halbfesten Zubereitung in der Form einer Salbe, die Octenidin-dihydrochlorid enthält, bei dem:
i) Octenidin-dihydrochlorid in einem Lösungsmittel, ausgewählt aus 1,2-Propylenglykol, Glycerol und Mischungen daraus, aufgelöst wird, um eine Lösung mit 0,01 bis 5 Gewichts-% Octenidin-dihydrochlorid auf der Grundlage des Gewichts der Lösung (Phase I) herzustellen,
ii) ein Emulgator mit HLB<8 zusammen mit einer Lipidphase (Phase II) bereitgestellt wird,
iii) die Phasen I und II getrennt auf eine Temperatur von 50 °C bis 100 °C erwärmt werden,
iv) die Phase I in die Phase II so eingemischt wird, dass das Gewichtsverhältnis zwischen der Phase I und der Phase II von 1:99 bis 20:80 liegt und
v) die Mischung gegebenenfalls homogenisiert wird.

2. Verfahren nach Anspruch 1, **dadurch gekennzeichnet, dass** die Konzentration von Octenidin-dihydrochlorid in der in Schritt i) zubereiteten Lösung bei 0,02 bis 2 Gewichts-% auf der Grundlage des Gewichts der Lösung liegt.

3. Verfahren nach einem der vorstehenden Ansprüche, **dadurch gekennzeichnet, dass** das Gewichtsverhältnis zwischen Phase I und Phase II von 2:98 bis 15:85 liegt.

4. Verfahren nach einem der vorstehenden Ansprüche, **dadurch gekennzeichnet, dass** die Lipidphase Vaseline, flüssiges Paraffin, Triglyceride oder Mischungen daraus und einen oder mehrere Emulgatoren mit HLB-Wert <8, ausgewählt aus Glycerol-(mono-,di-,tri-) [Adipat, Alkanoat (C₈, C₁₀ und C₁₈), Isostearat], Polyglycerylestern, Sorbitanlaurat und Cetylstearylacetat und Mischungen daraus, umfasst.

## Revendications

1. Procédé pour préparer une préparation multiphase semi-solide sous la forme d'un onguent qui contient du dichlorhydrate d'octénidine, dans laquelle :
i) le dichlorhydrate d'octénidine est dissous dans un solvant choisi parmi le 1,2-propylèneglycol, le glycérol et leurs mélanges, de manière à préparer une solution de 0,01 % à 5 % en poids de dichlorhydrate d'octénidine sur la base du poids de la solution (phase I),
ii) un émulsionnant avec un HLB < 8 est fourni conjointement avec une phase lipidique (phase II),
iii) les phases I et II sont chauffées séparément à une température de 50 °C à 100 °C,
iv) la phase I est incorporée à la phase II de sorte que le rapport pondéral entre la phase I et la phase II se situe dans une plage de 1:99 à 20 :80 et,
v) le cas échéant, le mélange est homogénéisé.

2. Procédé selon la revendication 1, **caractérisé en ce que** la concentration de dichlorhydrate d'octénidine dans la solution préparée à l'étape i) se situe dans une plage de 0,02 à 2 % en poids sur la base du poids de la solution.

3. Procédé selon l'une quelconque des revendications précédentes, **caractérisé en ce que** le rapport pondéral entre la phase I et la phase II se situe dans une plage de 2 :98 à 15 :85.

4. Procédé selon l'une quelconque des revendications précédentes, **caractérisé en ce que** la phase lipidique comprend de la vaseline, de la paraffine liquide, des triglycérides ou leurs mélanges et un ou plusieurs émulsionnants avec une valeur HLB < 8 choisis parmi (mono, di, tri) [adipate, alcanoate (en C₈, C₁₀ et C₁₈), isostéarate] de glycérol, les poly(esters de glycéryle), le laurate de sorbitan et l'acétate de cétylstéaryle et leurs mélanges.
